# EUROPÄISCHE PATENTSCHRIFT

(11) **EP 2 623 020 B1**
(45) Veröffentlichungstag und Bekanntmachung des Hinweises auf die Patenterteilung: **07.06.2023**
(21) Anmeldenummer: 13153775.5
(22) Anmeldetag: 02.02.2013
(51) Int. Cl.: A61B 3/10, A61B 5/00

(54) **Vorrichtung zur Prüfung der Otolithenfunktion und Verfahren zur Bestimmung der subjektiven visuellen Vertikalen**
Device for testing the otolith function and method for determining the subjective visual verticals
Dispositif destiné à contrôler la fonction otolithique et procédé de détermination de verticales visuelles subjectives

(30) Priorität: 03.02.2012 DE 102012001981
(43) Veröffentlichungstag der Anmeldung: 07.08.2013
(73) Patentinhaber: Chronos Vision GmbH, 12247 Berlin (DE)
(72) Erfinder: Spasovski, Saso, 13088 Berlin (DE); Pogade, Wolfgang, 12247 Berlin (DE)
(74) Vertreter: Willems, Volker

(56) Entgegenhaltungen:
- JP-A- 2001 305 988
- US-A1- 2004 006 287
- MARS FRANCK ET AL: "Perception of the vertical with a head-mounted visual frame during head tilt.", ERGONOMICS 15 AUG 2004, Bd. 47, Nr. 10, 15. August 2004 (2004-08-15), XP002695738, ISSN: 0014-0139

## Beschreibung

Die Erfindung betrifft eine Vorrichtung zur Prüfung der Otolithenfunktion gemäß dem Oberbegriff von Patentanspruch 1 und ein Verfahren zur Bestimmung der Subjektiven Visuellen Vertikalen gemäß dem Oberbegriff von Patentanspruch 12.

Die Otolithenorgane im Gleichgewichtsorgan des Menschen tragen wesentlich zur korrekten Wahrnehmung der Körperlage im Raum bei. Sie werden durch Utrikulus und Sakkulus gebildet. Eine Störung der Otolithenorganfunktion kann zur Fehleinschätzung der Körperlage, zu Schwindel und zu einer Gangunsicherheit mit Fallneigung führen.

Die Otolithenorgane oder kurz Otolithen befinden sich im Kopf und funktionieren nach dem Prinzip eines Trägheitssensors. Bei einer linearen Beschleunigung werden die mit den Sinneshaaren verbundenen Otokonien, die aus Kristallen gebildet sind, leicht verschoben und lösen in den Sinneszellen Nervenerregungen aus. In normalen Alltagssituationen handelt es sich häufig um die Erdanziehungskraft, die bei Kippung des Kopfes auf die Otolithen wirkt und somit durch die Sinneszellen eine Nervenerregung auslöst. Durch die Otolithenorgene werden also im Alltag Kopfkippungen relativ zur Raumvertikalen detektiert. Dies stellt eine wichtige Information für die Koordinierung des Körpers und für die aufrechte Körperhaltung dar.

Bittet man beispielsweise eine Person im Dunkeln, also ohne visuelle Orientierungsmöglichkeit, eine sichtbare Leuchtleiste oder leuchtende Linie so einzustellen, dass sie entsprechend ihrer Empfindung lotrecht bzw. senkrecht im Raum steht, erhält man gut reproduzierbare Ergebnisse, die bei gesunden Menschen weitgehend der tatsächlichen Vertikalen im Raum, d.h. der Richtung der Gravitationskraft, entsprechen. Das Ergebnis dieser Messung wird als "Subjektive Visuelle Vertikale" (SW) bezeichnet und durch die Information der Otolithenorgane ermöglicht. Die SVV kennzeichnet somit die von einer Person subjektiv empfundene vertikale Orientierung bzw. die Oben-Unten-Orientierung im Raum. Somit können die Ergebnisse, die durch die Messung der SVV geliefert werden, zur nachfolgenden klinischen Prüfung der Otolithenfunktion verwendet werden.

In der Veröffentlichung von A.H. Clarke et. al., "Unilateral examination of utricle and saccule function", Journal of Vestibular Research 13 (2003) 215-225, wird ein Messsystem und ein Verfahren zur Bestimmung der Subjektiven Visuellen Vertikalen beschrieben, bei dem eine Person in einen Dom blickt, in dem eine Lichtlinie erscheint, während die Person zusammen mit dem Dom um die Körperachse rotiert. Die Person dreht nun die Lichtlinie so lange, bis sie diese als vertikal ausgerichtet empfindet.

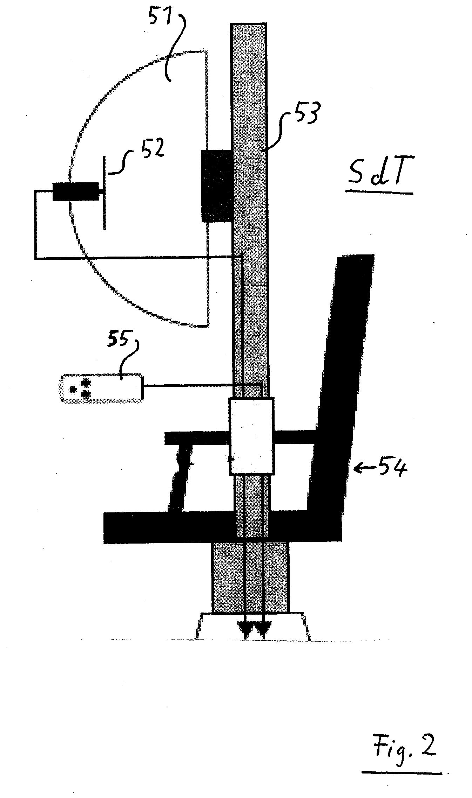

Figur 2 zeigt ein Messsystem zur Bestimmung der subjektiven visuellen Variablen nach dem oben genannten Stand der Technik. Es umfasst einen Dom 51 in Form einer Halbkugel, die einen Durchmesser von ca. 60 cm aufweist. In dem Dom 51 ist eine Reihe von LEDs linienartig angeordnet, so dass sie eine Lichtlinie 52 bilden, die mittels eines Motors drehbar ist. Der Dom 51 ist durch ein Rahmengestell 53 fest mit einem Drehstuhl 54 verbunden, auf dem eine Person Platz nimmt. Bei der Messung dreht sich die gesamte Anordnung einschließlich der Person um eine senkrechte Drehachse, die ca. 3,5 cm seitlich zum Zentrum des Kopfes versetzt ist, um eine Zentrifugalkraft entweder auf das linke oder auf das rechte Gleichgewichtsorgan auszuüben. Mittels einer Bedienungseinheit 55 dreht die Person die Lichtlinie 52 nun so lange, bis sie diese subjektiv als vertikal ausgerichtet wahrnimmt. Die Messung erfolgt bei verschiedenen Drehgeschwindigkeiten, die unterschiedlichen Neigungen des Kopfes entsprechen. Anstatt einer Drehung zur Stimulierung der Gleichgewichtsorgane kann auch eine Neigung erfolgen, indem die Anordnung mit der darin fixierten Person gekippt wird.

Ein Nachteil dieser Anordnung besteht darin, dass sie sehr groß ist. Weiterhin muss die Messung in einem vollkommen abgedunkelten Raum erfolgen, um jegliche Orientierungsmöglichkeit durch Objekte oder durch Licht außerhalb des Doms auszuschließen. Es ist weiterhin notwendig, die jeweilige Person in einen speziell ausgerichteten Untersuchungsraum zu bringen und in der Messvorrichtung zu positionieren und zu fixieren.

Die Europäische Patentanmeldung EP 0 363 521 A1 beschreibt eine Einrichtung zur Funktionsprüfung der Otolithen, die eine auf dem Kopf einer Person aufsetzbare, abgedunkelte Brille mit einem Neigungssensor umfasst. Vor dem Auge der Person befindet sich am Brillengestell ein Messeinsatz, durch den über einen Spalt mittels einer intensitätsstarken Strahlung bzw. einem Blitz eine erste Leuchtlinie dargeboten wird, die auf der Retina der Versuchsperson ein strichförmiges Nachbild erzeugt. Bei einer Neigung des Kopfes wird über denselben Spalt eine zweite Leuchtlinie mittels einer intensitätsschwachen Strahlung erzeugt. Die Person dreht nun mittels einer Verstellklappe an der Brille den Spalt und damit die mit der intensitätsschwachen Strahlung erzeugte zweite Leuchtlinie so lange, bis diese mit dem Nachbild der ersten Leuchtlinie auf der Retina zur Deckung kommt.

US 2004/006287 beschreibt ein Vertigo-Managementsystem und -verfahren, das eine Raummanövriervorrichtung, vorzugsweise unter Computersteuerung verwendet, um eine Person unterschiedlich im Raum auszurichten und somit eine vestibulare Aktivität zu erzeugen, die direkt mit der räumlichen Bewegung in Beziehung steht. Mit Beschleunigungssensoren werden Daten zur räumlichen Orientierung und Drehbewegung erzeugt. Dabei trägt die Person eine Maske, in der mit Infrarotkameras Augenbewegungen aufgenommen werden. Es werden Daten gesammelt, die sich auf die Orientierung und die Aktivität beziehen, zusammen mit bestimmten Reaktionsdaten und Videokameradaten.

In MARS FRANCK ET AL: "Perception of the vertical with a head mounted visual frame during head tilt", ERGONOMICS 15 AUG 2004, Bd47, Nr. 10, 15. August 2004 (2004-08-15), XP002695738, ISSN:0014-0139 ist eine Studie beschrieben, die den Einfluss eines am Kopf einer Person befestigten Rahmens auf die Wahrnehmung der Vertikalen untersucht, wenn der Kopf geneigt wird, im Vergleich zur Wahrnehmung der Vertikalen, wenn der Rahmen erdbefestigt ist. Dabei tragen verschiedene Testpersonen ein Video Headset, um den Einfluss eines am Kopf einer Person fixierten, für die Person sichtbaren Rahmens auf deren Empfindung der Vertikalen bei einer Verkippung des Kopfes zu untersuchen.

JP 2001 305988 A beschreibt eine brillenartige Anzeigevorrichtung mit einem Paar von optischen Elementen. Die Anzeigevorrichtung steuert Anzeigeinhalte mittels Videodaten in einer Speicherkarte, und ändert die Anzeigeinhalte durch Austauschen der Speicherkarte.

Es ist die Aufgabe der Erfindung, ein Verfahren zur Bestimmung der subjektiven visuellen Vertikalen anzugeben, das einen größeren Einsatzbereich ermöglicht, dabei einen geringen Aufwand erfordert und schnell durchführbar ist. Weiterhin soll eine Vorrichtung zur Prüfung der Otolithenfunktion geschaffen werden, die flexibel einsetzbar ist und zudem klein ist und genaue Messergebnisse liefert.

Diese Aufgabe wird gelöst durch die Vorrichtung zur Prüfung der Otolithenfunktion gemäß Patentanspruch 1 und durch das Verfahren zur Bestimmung der subjektiven visuellen Vertikalen gemäß Patentanspruch 12. Weitere vorteilhafte Merkmale und Details ergeben sich aus den abhängigen Patentansprüchen, der Beschreibung, und den Zeichnungen.

Die erfindungsgemäße Vorrichtung zur Prüfung der Otolithenfunktion bei Personen umfasst eine Maske, die einer Person aufsetzbar ist; einen Neigungssensor, dessen Signale die Neigung der Maske und/oder eine auf die Maske wirkende Beschleunigung repräsentieren; ein elektronisches Display zur Darstellung eines Bildes innerhalb der Maske, ein Bediengerät zum Senden von Steuersignalen an die Maske; wobei das Bediengerät Bedienelemente umfasst, mit denen das auf dem Display dargestellte Bild im oder gegen den Uhrzeigersinn gedreht werden kann, um eine vertikal ausgerichtete Bildlage visuell einzustellen; wobei die Maske lichtundurchlässig ist und der Person lichtdicht aufsetzbar ist, der Neigungssensor in die Maske integriert ist, und das Bediengerät tragbar ist, so dass die Person in einer beliebigen Position die Bildlage manuell einstellen kann; und wobei eine drahtlose Datenübertragungseinrichtung mit einer bidirektionalen Funkstrecke zur Übertragung von Messdaten und der Steuersignale zwischen der Maske und dem Bediengerät und einer Auswerteeinheit vorgesehen ist.

Durch die erfindungsgemäße Vorrichtung kann mit relativ geringem Aufwand und mit großer Genauigkeit die subjektive visuelle Vertikale bestimmt und damit die Otolithenfunktion gemessen oder geprüft werden. Die durch die Erfindung erzielte kleine und leichte Bauweise erlaubt es, die Messung an dem Ort durchzuführen, an dem sich die Person gerade befindet. Es ist keine Positionierung und keine Fixierung der Person in einer speziellen Messapparatur erforderlich, so dass beispielsweise Messungen auch an bettlägerigen oder gebrechlichen Personen durchgeführt werden können. Weiterhin ist keine Verdunkelung des Raumes erforderlich, in dem sich die Person bei der Messung befindet.

Der Begriff "vertikal ausgerichtet" oder "vertikal ausgerichtete Bildlage" soll so verstanden werden, dass die Person, die die Maske trägt, allein durch das Betrachten des Bildes und ohne sonstige Bezugspunkte die Bildlage oder die Lage eines abgebildeten Objekts im Raum subjektiv als vertikal ausgerichtet empfindet. Das heißt, für die Person entspricht in diesem Fall "oben" und "unten" im Bild auch exakt "oben" und "unten" im Raum. Die vertikal ausgerichtete Bildlage wird dabei auch als gleichbedeutend mit einer horizontalen Ausrichtung angesehen, zum Beispiel im Fall eines sich waagerecht erstreckenden Bildobjekts oder Bildes, und soll diese begrifflich mit umfassen. Subjektive vertikale Ausrichtung heißt allgemein, dass eine Vertikale bzw. eine Horizontale im Bild vom Betrachter exakt als vertikal bzw. horizontal im Raum ausgerichtet wahrgenommen wird.

Vorteilhafterweise erzeugt das Bediengerät die Steuersignale für das Display. Die Steuersignale bewirken ein Drehen des Bildes auf dem Display um die Betrachtungsrichtung, das heißt bei aufgesetzter Maske um die Blickrichtung der Person, die die Maske trägt. Dadurch kann die Person in einer beliebigen und damit entspannten Position die Bildlage während der Messung subjektiv vertikal bzw. horizontal ausgerichtet manuell einstellen, ohne dass sie dazu den Arm in eine bestimmte Position bringen muss.

Bevorzugt umfasst die Vorrichtung ein Spiegelsystem, das innerhalb der Maske derart angeordnet ist, dass es das auf dem Display erscheinende Bild zum Betrachter hin umlenkt bzw. reflektiert. Dadurch wird eine besonders kompakte Bauform erreicht so dass die Vorrichtung besonders leicht und flexibel einsetzbar ist.

Vorteilhafterweise vergleicht die Auswerteeinheit bei einer als vertikal ausgerichtet erscheinenden bzw. empfundenen Bildlage den Kippwinkel des Bildes relativ zur Maske mit dem Signal des Neigungssensors. Hierdurch ist es möglich, automatisch eine Abweichung der Differenz zwischen der subjektiv empfundenen Vertikalen und der tatsächlichen Vertikalen gegenüber zuvor eingestellten Normwerten zu ermitteln und anzuzeigen.

Der Neigungssensor ist in der Maske integriert und zum Beispiel als dreidimensionaler Beschleunigungssensor ausgestaltet. Dadurch wird die genaue Lage des Kopfes im Raum erfasst und es können Neigungen des Kopfes oder Beschleunigungen, die auf den Kopf wirken, in allen drei Raumrichtungen bei der Messung berücksichtigt werden. Die Messung und die Ermittlung der subjektiven visuellen Vertikalen kann also bei verschiedenen Neigungsrichtungen des Kopfes durchgeführt werden, beispielsweise wenn die Person bei der Messung liegt. In diesem Fall ist der Kopf nicht vertikal ausgerichtet, während die Kopfneigung zur Seite erfolgt.

Die Maske ist insbesondere kopffest vor den Augen positionierbar bzw. montierbar und dadurch bei der Messung starr mit dem Kopf und damit auch mit dem im Innenohr befindlichen Gleichgewichtsorgan verbunden. Durch die Kopfmontage sind beliebige Testbedingungen möglich, beispielsweise auf einem Kipptisch, einem Drehstuhl oder einem Schlitten zur linearen Beschleunigung. Die Messungen können an beliebigen Orten und in beliebiger Körperlage durchgeführt werden, beispielsweise zu Hause oder auch während die Person im Bett liegt oder in einem Rollstuhl sitzt. Zur Messung muss der Person lediglich die Maske aufgesetzt werden.

Insbesondere kann das Bild als Lichtmuster und/oder als Lichtlinie ausgestaltet sein. Dies erleichtert dem Betrachter die subjektive vertikale Ausrichtung des Bildes und führt somit zu einer höheren Messgenauigkeit. Dabei kann die Lichtlinie sich vertikal oder horizontal im Bild erstrecken. Wie oben erwähnt, wird unter vertikaler Ausrichtung auch die horizontale oder waagerechte Ausrichtung zum Beispiel einer horizontalen Linie oder allgemein eines Objekts im Bild verstanden.

Das Bild kann beispielsweise auch als Bildfolge oder als Video ausgestaltet sein. Dadurch werden weitergehende Untersuchungen ermöglicht, beispielsweise die Wirkung von visuellen Wahrnehmungen oder Filmen auf das Gleichgewichtsorgan oder allgemein optokinetische Tests.

Vorteilhafterweise ist in der Maske ein Bildspeicher integriert. Das Bild oder die Bilder können vorteilhaft auch im Handgerät oder auf einem Mini-PC, der sich bei der Messung im Schoss der Person befindet, gespeichert sein. Durch diese Maßnahmen werden Übertragungsfehler und dadurch verursachte mögliche Störungen im Bild minimiert, und die erforderliche Bandbreite ist extrem niedrig.

Bevorzugt ist vor dem Display ein Diffusorelement angeordnet, das zum Beispiel als Diffusorscheibe ausgestaltet ist. Dadurch wird eine Orientierungsmöglichkeit des Betrachters an Bildpixeln oder an treppenförmigen Pixelmustern bei schräg eingestellten Bildlinien verhindert, wodurch die Messergebnisse noch weiter verbessert werden.

Insbesondere kann das auf dem Display dargestellte Bild derart gestaltet sein, dass es frei von Kanten ist, wobei das Bild zum Beispiel künstlich verrauscht ist und/oder ausschließlich irreguläre bzw. unregelmäßige oder gekrümmte Strukturen aufweist. Die Lichtlinie oder Linie kann zum Beispiel aus einzelnen Kreisen gebildet sein, um Kanten auszuschließen. Auch durch diese Maßnahmen wird eine Orientierungsmöglichkeit des Betrachters an Bildpixeln oder an treppenförmigen Pixelmustern bei schräg eingestellten Bildlinien verhindert.

Die drahtlose Datenübertragungseinrichtung mit der bidirektionalen Funkstrecke dient zum Austausch von Messdaten und Steuersignalen. Dieser Austausch erfolgt zwischen der Maske und dem Bediengerät und der Auswerteeinheit. Dadurch kann die Messung beispielsweise auch erfolgen, während die Person rotiert wird, ohne dass aufwendige elektrische Verbindungen zwischen der Maske und der Auswerteeinheit notwendig sind.

Bevorzugt umfasst die Vorrichtung einen 3-achsigen Drehratensensor, der vorteilhafterweise in der Maske integriert ist. Dadurch können bei Messungen der SVV auf einem Drehstuhl die zusätzlich auftretenden Beschleunigungen eliminiert werden.

Das erfindungsgemäßen Verfahren dient zur Bestimmung der subjektiv wahrgenommenen visuellen Vertikalen bei Personen und umfasst die Schritte: Darstellen eines Bildes auf einem elektronischen Display innerhalb der Maske innerhalb einer Maske, die einer Person aufgesetzt wird; Erfassen der Neigung des Kopfes und/oder einer auf den Kopf wirkenden Beschleunigung mit einem Neigungssensor; wobei das Bild mittels Steuersignalen um die Betrachtungsrichtung gedreht wird, bis es der Person als vertikal im Raum ausgerichtet erscheint; wobei die Maske lichtundurchlässig ist und der Person derart aufgesetzt wird, dass kein Licht von außen in den Innenraum der Maske gelangt, der Neigungssensor in die Maske integriert ist, und die Person mit einem tragbaren Bediengerät die subjektiv wahrgenommene vertikale Bildlage selbst manuell einstellt, während sie sich in einer beliebigen Position befindet; und wobei Messdaten und die Steuersignale mit einer bidirektionalen Funkstrecke zwischen der Maske und dem Bediengerät und einer Auswerteeinheit übertragen werden.

Durch das erfindungsgemäße Verfahren kann schnell und mit relativ geringem Aufwand die Subjektive Visuelle Vertikale einer Person bestimmt und damit die Otolithenfunktion der Person gemessen oder geprüft werden. Die erhaltenen Ergebnisse sind sehr genau, da über die visuelle, subjektive "Oben-Unten-Ausrichtung" des Bildes durch die zu untersuchende Person direkt das subjektive Empfinden der Richtung der Erdanziehungskraft oder allgemein einer Beschleunigung erfasst wird. Die Messung kann an einem beliebigen Ort durchgeführt werden, beispielsweise in der Wohnung der Person, da keine große Messapparatur und kein abgedunkelter Raum erforderlich ist.

Bevorzugt stellt die Person mit dem tragbaren Bediengerät die subjektiv wahrgenommene vertikale Bildlage selbst ein.

Vorteilhaft dreht die Person bei einer definierten Kopfneigung oder Beschleunigung das Bild um die Blickrichtung in die Lage, in der es ihr als vertikal im Raum ausgerichtet erscheint, wobei bei der so eingestellten Bildlage der Kippwinkel des Bildes relativ zur Maske erfasst und mit dem Signal des Neigungssensors verglichen wird.

Insbesondere kann als Bild ein Lichtmuster und/oder eine Lichtlinie dargestellt werden. Es ist aber für bestimmte Untersuchungen auch vorteilhaft, dass als Bild eine Bildfolge oder ein Video dargestellt wird.

Je nach dem Zweck der Messung kann die Person während der Messung beschleunigt werden, beispielsweise durch Rotation oder auch durch eine lineare Beschleunigung in einer Richtung im Raum.

Bevorzugt wird für das erfindungsgemäße Verfahren eine erfindungsgemäße Vorrichtung verwendet. Die oben im Zusammenhang mit der erfindungsgemäßen Vorrichtung beschriebenen Vorteile gelten auch für das erfindungsgemäße Verfahren und umgekehrt.

Nachfolgend wird die Erfindung anhand der Zeichnungen beispielhaft beschrieben. Es zeigen:
- **Fig. 1**: eine Vorrichtung gemäß einer bevorzugten Ausführungsform der Erfindung in schematischer Darstellung von der Seite;
- **Fig. 2**: eine Vorrichtung zur Messung der Subjektiven Visuellen Variablen gemäß dem Stand der Technik;
- **Fig. 3a und 3b**: schematisch den Strahlengang in der erfindungsgemäßen Vorrichtung, wie sie in Figur 1 gezeigt ist, in einer Ansicht von oben bzw. von der Seite;
- **Fig. 4**: schematisch die Anordnung der optischen Elemente in einer Vorrichtung gemäß einer zweiten Ausführungsform der Erfindung;
- **Fig. 5**: eine schematische Darstellung eines erfindungsgemäßen Messystems;
- **Fig. 6**: schematisch eine Maske mit einem Display und einem Handgerät gemäß der Erfindung zur Erläuterung des erfindungsgemäßen Verfahrens;

- **Fig. 7**: eine Grafik, die beispielhaft Ergebnisse einer Bestimmung der SVV gemäß der Erfindung zeigt; und
- **Fig. 8a und 8b**: schematisch eine beispielhafte Lichtlinie in einem zufällig gekippten Zustand (Fig. 8a) und in einem subjektiv vertikal ausgerichteten Zustand (Fig. 8b).

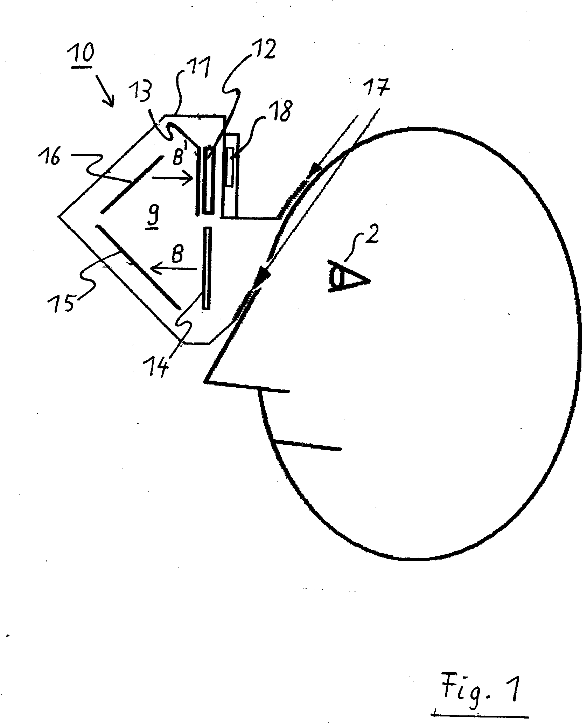

**Figur 1** zeigt schematisch eine Vorrichtung zur Prüfung der Otolithenfunktion gemäß einer bevorzugten Ausführungsform der Erfindung. Die Vorrichtung umfasst eine Maske 10 mit einem lichtundurchlässigen Gehäuse 11, die einer Person zum Zweck der Messung derart aufgesetzt wird, dass sie sich im Bereich des Gesichts vor den Augen 2 der Person befindet. Im aufgesetzten Zustand ist die Maske 10 lichtdicht und starr mit dem Kopf verbunden, das heißt, es kann kein Licht von außen in den Innenraum 9 der Maske 10 und somit in das Auge 2 gelangen.

In der Maske 10 ist ein Neigungssensor 18 integriert. Der Neigungssensor 18 ist am lichtdichten Gehäuse 11 befestigt und als dreidimensionaler Beschleunigungssensor ausgestaltet. Er liefert Signale, welche die jeweilige Neigung des Kopfes oder allgemein eine auf den Kopf wirkende Beschleunigung repräsentieren. Weiterhin befindet sich in der Maske 10 integriert ein elektronisches Display 12, das zur Darstellung eines Bildes innerhalb der Maske 10 dient und für die Person, welche die Maske 10 trägt, sichtbar ist. Das Display 12 wird durch entsprechende Steuersignale derart angesteuert, dass sich das dargebotene Bild um die Betrachtungsrichtung B, B' dreht. Auf diese Weise wird das Bild für den Betrachter vertikal ausgerichtet, das heißt, bei der Messung wird die Bildlage durch Drehen um die Betrachtungsrichtung derart eingestellt, dass der Betrachter sie subjektiv als vertikal im Raum ausgerichtet empfindet.

Die Maske 10 ist in Form einer Brille gestaltet. Um die Lichtdichtigkeit der Brille bzw. Maske 10 im aufgesetzten Zustand zu gewährleisten, ist ein elastisches Element 17 als Gesichtsanschluss vorgesehen. Das elastische Element 17 ist beispielsweise aus einem dunklen, lichtundurchlässigen Schaumstoff, Gummi oder ähnlichem gebildet. Es befindet sich an dem Rand der Maske 10, der den Kontakt zur Gesichtsfläche bildet.

Im Innenraum 9 der Maske 10 ist eine Spiegelvorrichtung angeordnet, die aus einem Hauptspiegel 15 und einem Displayspiegel 16 besteht. Die beiden als Oberflächenspiegel ausgestalteten Spiegel 15 und 16 sind derart angeordnet, dass das auf dem Display 12 dargebotene Bild oder Lichtmuster zum Auge 2 des Betrachters bzw. der zu untersuchenden Person gelenkt wird. Das Display 12 ist dabei im Strahlengang auf der Seite der Öffnung des Gehäuses 11 angeordnet, durch welche die Person in den Innenraum 9 der Maske 10 blickt. Dagegen sind die beiden Spiegel 15, 16 auf der gegenüberliegenden Seite angeordnet, so dass sie das Bild auf dem Display 12 zum Auge 2 des Betrachters hin zurückreflektieren, wobei der Strahlengang vom Display 12 zur Spiegelvorrichtung 15, 16 parallel zum Strahlengang zwischen der Spiegelvorrichtung 15, 16 und dem Auge 2 des Betrachters verläuft.

Vor dem Display 12 ist ein Diffusorelement 13 angeordnet, das vorzugsweise flächig oder scheibenförmig ausgestaltet ist. Das Diffusorelement 13 verhindert eine Orientierung des Betrachters an Bildpixeln, die zum Beispiel bei der Darstellung von schräg im Bild verlaufenden Linien als treppenförmige Muster sichtbar werden. Durch das Diffusorelement 13 ist es ausgeschlossen, dass der Betrachter Rückschlüsse auf die tatsächliche vertikale Lage eines Bildelements im Raum ziehen kann.

Zwischen der Betrachtungsöffnung des Gehäuses 11 und der Spiegelvorrichtung befindet sich eine Fresnellinse 14 zur Fokussierung des Bildes.

**Figur 2** zeigt die aus dem Stand der Technik bekannte Messvorrichtung mit einem höhenverstellbaren Dom 51, die im Einleitungsteil bereits diskutiert wurde.

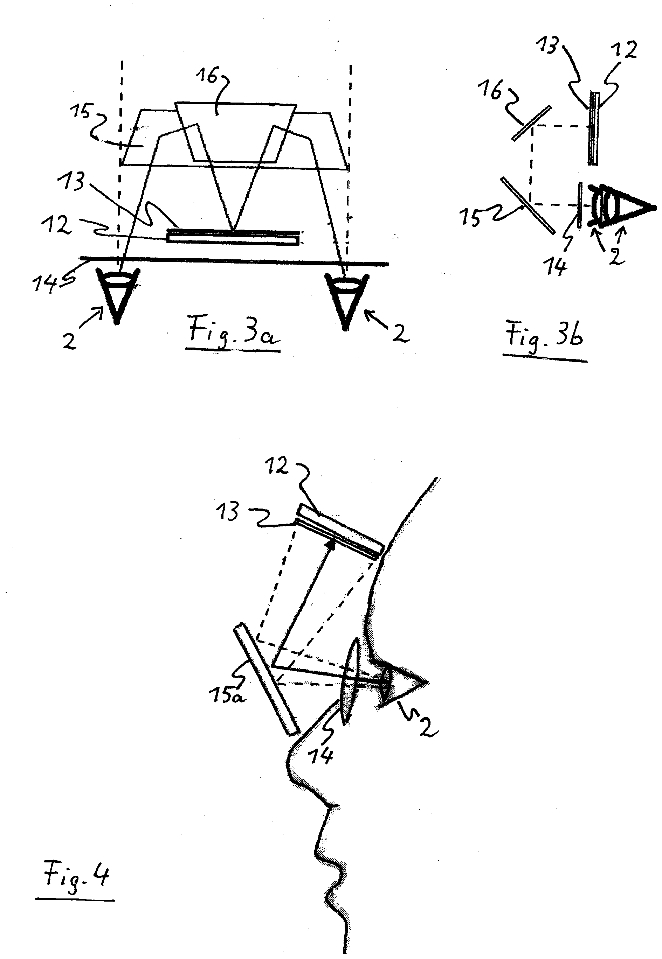

Die **Figuren 3a und 3b** zeigen zur Verdeutlichung den Strahlengang in der erfindungsgemäßen Vorrichtung gemäß der ersten Ausführungsform nochmals schematisch in einer Draufsicht von oben (Figur 3a) und von der Seite (Figur 3b). Ausgehend vom Display 12 mit dem davor angeordneten Diffusorelement 13 wird das Bild über die gegenüberliegende Spiegelvorrichtung mit dem Displayspiegel 16 und dem Hauptspiegel 15 und anschließend durch die Fresnellinse 14 zum Auge 2 des Betrachters geführt.

**Figur 4** zeigt die Anordnung von Display und Spiegelvorrichtung in einer Vorrichtung gemäß einer zweiten Ausführungsform der Erfindung. Dabei befindet sich das Display 12 ebenfalls in der Maske oberhalb der Betrachtungsöffnung, das heißt, bei aufgesetzter Maske oberhalb des Auges 2 des Betrachters. Jedoch ist die Displayfläche nicht parallel zum Auge des Betrachters bzw. zur Linse des Auges, wie bei der in Figur 1 gezeigten Vorrichtung, sondern schräg dazu angeordnet. Die Spiegelvorrichtung besteht in diesem Fall aus nur einem Spiegel 15a, der das oberhalb des Auges 2 auf dem Display 12 dargestellte Bild zum Auge 2 hin reflektiert.

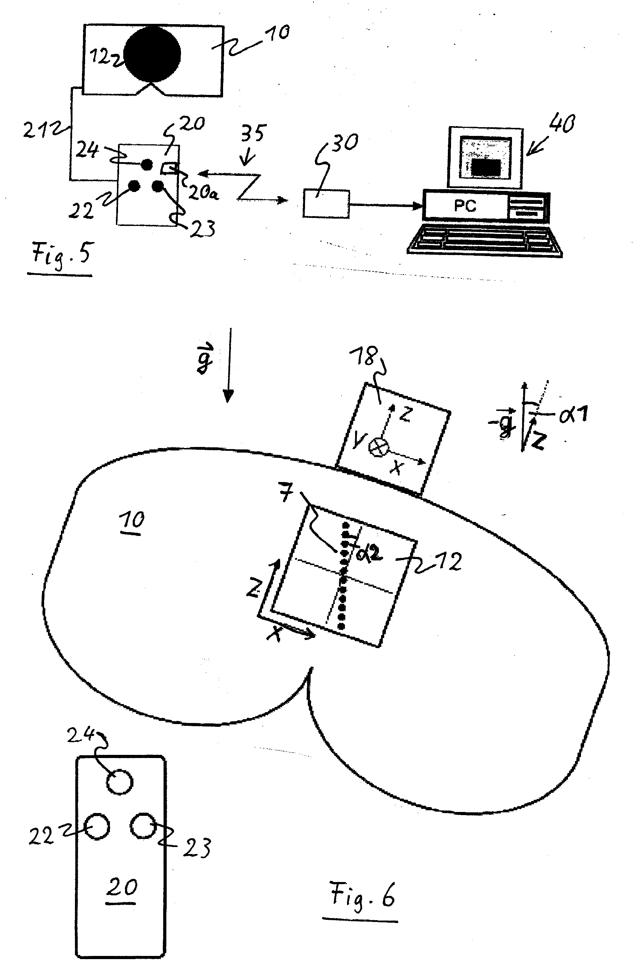

**Figur 5** zeigt die erfindungsgemäße Vorrichtung als ein Messsystem, das zusätzlich zur Maske 10 ein tragbares Bediengerät 20, eine Datenübertragungseinheit 30 und eine Auswerteeinheit 40 umfasst.

Das Bediengerät 20 ist durch eine elektrische Verbindung 21 in Form eines Kabels mit der Messbrille, das heißt mit der Maske 10, verbunden. Es umfasst die Stromversorgung für die Messbrille sowie Bedienelemente 22 und 23, mit denen das auf dem Display 12 innerhalb der Maske dargebotene Bild oder Lichtmuster im Uhrzeigersinn bzw. gegen den Uhrzeigersinn gedreht werden kann. Das Bediengerät sendet Steuersignale an die Maske 10, welche das Drehen des auf dem Display 12 dargestellten Bildes, das beispielsweise eine Punktreihe ist, bewirken.

Ein weiteres Bedienelement 24 des Bediengerätes 20 dient zur Bestätigung, dass das Bild auf dem Display nach dem erfolgten Drehen als vertikal ausgerichtet empfunden wird. In diesem Fall wird durch Betätigung des Bedienelements 24 ein Bestätigungssignal ausgelöst und an die Auswerteeinheit 40 gesendet.

Über eine bidirektionale Funkstrecke 35, die eine drahtlose elektrische Verbindung zwischen der Maske 10, dem Bediengerät 20 und der Datenübertragungseinheit 30 bildet, werden Mess- und/oder Steuerdaten zwischen der Maske 10, dem Bediengerät 20 und der Auswerteeinheit 40 übertragen. Zu diesem Zweck umfasst das Bediengerät 20 ebenfalls eine Datenübertragungseinheit 20a. Die Datenübertragungseinheit 30 ist über einen USB Anschluss mit der Auswerteeinheit 40 verbunden, die beispielsweise durch eine Rechnereinheit oder einen PC mit einer entsprechenden Auswertesoftware gebildet wird.

Nachfolgend wird anhand von **Figur 6** beispielhaft der Ablauf einer Messung der Subjektiven Visuellen Vertikalen oder SVV mit der erfindungsgemäßen Vorrichtung erläutert.

Die Maske 10 wird einer Person aufgesetzt, deren SVV bestimmt werden soll. Da die Maske 10 lichtdicht ist, gelangt nun kein Licht mehr in die Augen der Person, sodass diese keinerlei optische Orientierung hat.

Anschließend wird eine Lichtlinie 7 als Bild auf dem Display 12 dargestellt, die zu Beginn jeder Messung in einem Zufallswinkel ausgerichtet ist. Nur die Lichtlinie 7 ist innerhalb der Maske 10 für die Testperson sichtbar.

Die Person wird nun angewiesen, mit Hilfe des tragbaren Bediengeräts 20 die Lichtlinie zu drehen, so dass diese entsprechend der empfundenen vertikalen Gravitationskraft g ausgerichtet ist.

Sobald diese subjektiv empfundene vertikale Ausrichtung der Linie 7 eingestellt ist, drückt die Testperson den Bestätigungsknopf auf dem Bediengerät 20, das heißt das Bedienelement 24.

Zur Erhöhung der Messgenauigkeit werden üblicherweise mehrere Messungen durchgeführt, beispielsweise drei bis sechs. Zwischen den einzelnen Messungen wird die Lichtlinie 7 auf dem Display 12 ausgeschaltet und dann erneut in einer anderen zufällig ausgerichteten Position wieder eingeschaltet. Die einzelnen Messergebnisse werden abgespeichert.

Die Subjektive Visuelle Vertikale ist als der eingestellte Winkel α2 der Lichtlinie 7 auf dem Display 12 definiert, das heißt, der Winkel zwischen der Lichtlinie 7 und der z-Richtung bzw. Vertikalrichtung des Displays 12 bzw. der Maske 10. Falls die Maske 10 und damit der Kopf exakt vertikal ausgerichtet sind, sollte die SVV bzw. der Winkel α2 im Normalfall Null Grad betragen. Bei einer Kippung des Kopfes sollte sich der Winkel α2 zwischen der subjektiv von der Testperson eingestellten Lichtlinie 7 und der z-Achse der Maske 10 dem Betrag nach entsprechend erhöhen und im Idealfall bei einer Verkippung des Kopfes um 90 Grad ebenfalls 90 Grad erreichen.

Die Messung wird bei verschiedenen Neigungen durchgeführt. Mit dem oben an der Maske 10 befestigten Neigungssensor 18 wird dabei zu jeder gemessenen Subjektiven Visuellen Vertikalen α2 die tatsächliche Neigung der Maske 10 gegenüber der Richtung der Gravitationskraft g als Neigungswinkel α1 gemessen. In der Auswerteeinheit werden die bei jeder Messung erhaltenen Winkel α2 und α1 miteinander verglichen. Anschließend können die Differenzen zwischen α2 und α1 mit Normwerten für bestimmte Personengruppen verglichen werden.

Im allgemeinen wird die Messfolge bei einer aufrechten Position der Person sowie bei symmetrisch nach links bzw. rechts gekippten Positionen durchgeführt. Optional ist es auch möglich, die Messung auf einem Drehstuhl durchzuführen. In diesem Fall kann eine unilaterale Otolithenstimulation erfolgen, indem die Rotationsachse entweder durch das linke oder durch das rechte Gleichgewichtsorgan verläuft, und somit nur das jeweils andere, außerhalb der Rotationsachse gelegene Otolithenorgan die Rotationskraft erfährt.

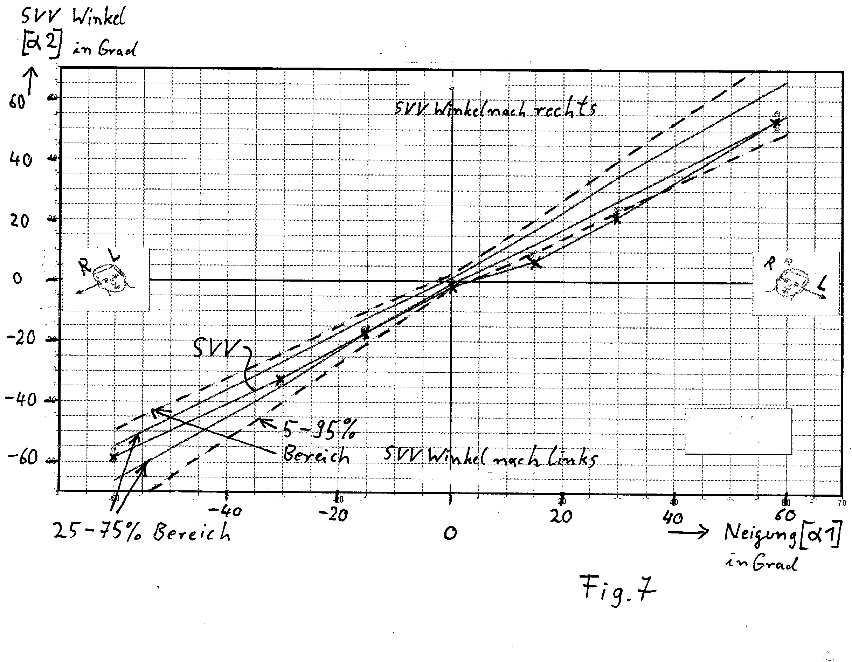

**Figur 7** zeigt ein Beispiel für die erhaltenen Messergebnisse. Die x-Achse bezeichnet den bei jeder Messung erhaltenen Neigungswinkel α1, der mit dem Neigungssensor ermittelt wurde. Die y-Achse bezeichnet die jeweils dazu ermittelten SVV Werte α2. Die bei bestimmten Neigungswinkeln α1 ermittelten SVV Werte α2 sind durch Kreuze markiert, die durch eine durchgezogene Linie (SW-Linie) verbunden sind. Die gestrichelten Linien zeigen die Grenzen des 5-95 % Normbereichs. Die durchgezogenen Linien zeigen die Grenzen des 25-75 % Normbereichs.

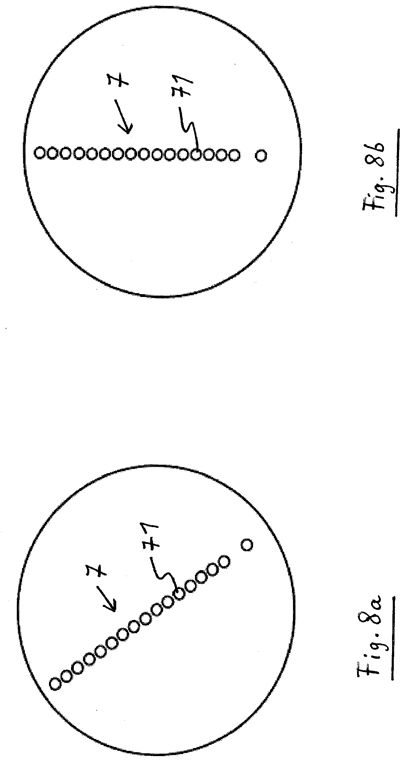

Die **Figuren 8a und 8b** zeigen in Ergänzung zu Fig. 6 und der dazugehörigen Beschreibung ein Bild, wie es bei der Messung der SW auf dem Display 12 erscheint. Das Bild ist als Lichtlinie 7 ausgestaltet, wobei die Lichtlinie 7 wiederum aus einzelnen kreisförmigen Bildelementen 71 gebildet ist, die in einer gerade verlaufenden Reihe angeordnet sind. Dies hat den Vorteil, dass im Bild keine treppenförmigen Pixelmuster zu erkennen sind, die oftmals an schräg verlaufenden Kanten entstehen. Wie oben bereits erläutert, wird zu Beginn der Messung die Linie 7 in einer schrägen Lage dargestellt, wobei der Winkel gegenüber der z-Achse im Koordinatensystem der Brille zufällig eingestellt ist (Fig. 8a). Die Testperson wird dann gebeten, mit Hilfe des Bedienelements 20 die leuchtende Linie 7 parallel zum Vektor der Gravitation einzustellen, das heißt, die Linie durch Rotation nach links oder nach rechts mit Hilfe der Bedienelemente 22 und 23 in die subjektiv wahrgenommene vertikale Lage zu bringen (Fig. 8b).

## Patentansprüche

1. Vorrichtung zur Prüfung der Otolithenfunktion bei Personen, umfassend:
eine Maske (10), die einer Person aufsetzbar ist;
einen Neigungssensor (18), dessen Signale die Neigung der Maske (10) und/oder eine auf die Maske (10) wirkende Beschleunigung repräsentieren;
ein elektronisches Display (12) zur Darstellung eines Bildes (7) innerhalb der Maske (10),
ein Bediengerät (20) zum Senden von Steuersignalen an die Maske (10),
wobei das Bediengerät (20) Bedienelemente (22, 23) umfasst, mit denen das auf dem Display (12) dargestellte Bild (7) im oder gegen den Uhrzeigersinn gedreht werden kann, um eine vertikal ausgerichtete Bildlage visuell einzustellen,
**dadurch gekennzeichnet, dass**
die Maske (10) lichtundurchlässig ist und der Person lichtdicht aufsetzbar ist,
dass der Neigungssensor (18) in die Maske (10) integriert ist, dass das Bediengerät (20) tragbar ist, so dass die Person in einer beliebigen Position die Bildlage manuell einstellen kann,
und dass eine drahtlose Datenübertragungseinrichtung (30) mit einer bidirektionalen Funkstrecke (35) zur Übertragung von Messdaten und der Steuersignale zwischen der Maske (10) und dem Bediengerät (20) und einer Auswerteeinheit (40) vorgesehen ist.

2. Vorrichtung nach Anspruch 1, **dadurch gekennzeichnet, dass** das Bediengerät (20) die Steuersignale für das Display (12) erzeugt.

3. Vorrichtung nach Anspruch 1 oder 2, **gekennzeichnet durch** ein Spiegelsystem (15, 16; 15a), das innerhalb der Maske (10) derart angeordnet ist, dass es das auf dem Display (12) erscheinende Bild zum Betrachter hin umlenkt.

4. Vorrichtung nach einem der vorhergehenden Ansprüche, **dadurch gekennzeichnet, dass** die Auswerteeinheit (40) bei einer als vertikal ausgerichtet erscheinenden Bildlage den Kippwinkel (α2) des Bildes relativ zur Maske (10) mit dem Signal des Neigungssensors (18) vergleicht.

5. Vorrichtung nach einem der vorhergehenden Ansprüche, **dadurch gekennzeichnet, dass** der Neigungssensor (18) als dreidimensionaler Beschleunigungssensor ausgestaltet ist.

6. Vorrichtung nach einem der vorhergehenden Ansprüche, **dadurch gekennzeichnet, dass** das Bild (7) als Lichtmuster und/oder als Lichtlinie ausgestaltet ist.

7. Vorrichtung nach einem der vorhergehenden Ansprüche, **dadurch gekennzeichnet, dass** das Bild (7) als Bildfolge oder als Video ausgestaltet ist.

8. Vorrichtung nach einem der vorhergehenden Ansprüche, **gekennzeichnet durch** einen in der Maske (10) integrierten Bildspeicher.

9. Vorrichtung nach einem der vorhergehenden Ansprüche, **dadurch gekennzeichnet, dass** vor dem Display ein Diffusorelement (13) angeordnet ist.

10. Vorrichtung nach einem der vorhergehenden Ansprüche, **dadurch gekennzeichnet, dass** das auf dem Display dargestellte Bild frei von Kanten ist, wobei das Bild insbesondere künstlich verrauscht ist und/oder ausschließlich irreguläre Strukturen aufweist.

11. Vorrichtung nach einem der vorhergehenden Ansprüche, **gekennzeichnet durch** einen 3-achsigen Drehratensensor.

12. Verfahren zur Bestimmung der Subjektiven Visuellen Vertikalen bei Personen, mit den Schritten:
Darstellen eines Bildes (7) auf einem elektronischen Display innerhalb einer Maske (10), die einer Person aufgesetzt wird; und
Erfassen der Neigung des Kopfes und/oder einer auf den Kopf wirkenden Beschleunigung mit einem Neigungssensor (18);
wobei das Bild (7) mittels Steuersignalen um die Betrachtungsrichtung B, B' gedreht wird, bis es der Person als vertikal im Raum ausgerichtet erscheint,
**dadurch gekennzeichnet,**
**dass** die Maske (10) lichtundurchlässig ist und der Person derart aufgesetzt wird, dass kein Licht von außen in den Innenraum (9) der Maske (10) gelangt,
der Neigungssensor (18) in die Maske (10) integriert ist,
und die Person mit einem tragbaren Bediengerät (20) die subjektiv wahrgenommene vertikale Bildlage selbst manuell einstellt, während sie sich in einer beliebigen Position befindet,
und Messdaten und die Steuersignale mit einer bidirektionalen Funkstrecke (35) zwischen der Maske (10) und dem Bediengerät (20) und einer Auswerteeinheit (40) übertragen werden.

13. Verfahren nach Anspruch 12, **dadurch gekennzeichnet, dass** die Person bei einer definierten Kopfneigung oder Beschleunigung das Bild (7) um die Blickrichtung B, B' in eine Lage dreht, in der es für die Person als vertikal im Raum ausgerichtet erscheint; und bei der so eingestellten Bildlage der Kippwinkel des Bildes (7) relativ zur Maske (10) erfasst und mit dem Signal des Neigungssensors (18) verglichen wird.

14. Verfahren nach Anspruch 12 oder 13, **dadurch gekennzeichnet, dass** als Bild (7) ein Lichtmuster und/oder eine Lichtlinie dargestellt wird.

15. Verfahren nach einem der Ansprüche 12 bis 14, **dadurch gekennzeichnet, dass** als Bild (7) eine Bildfolge oder ein Video dargestellt wird.

16. Verfahren nach einem der Ansprüche 12 bis 15, **dadurch gekennzeichnet, dass** die Person während der Messung beschleunigt wird.

## Claims

1. A device for testing the otolith function in persons, comprising:
a mask (10) which can be placed on a person;
an inclination sensor (18) whose signals represent the tilt of the mask (10) and/or an acceleration acting on the mask (10);
an electronic display (12) for displaying an image (7) within the mask (10),
an operating device (20) for sending control signals to the mask (10),
wherein the operating device (20) comprises operating elements (22, 23) with which the image (7) shown on the display (12) can be rotated clockwise or counterclockwise in order to visually adjust a vertically aligned image position,
**characterized in**
**that** the mask (10) is opaque and can be placed on the person in a light-tight manner,
**that** the inclination sensor (18) is integrated into the mask (10),
**that** the operating device (20) is portable so that the person can manually adjust the image position in any desired position, and
**that** a wireless data transmission device (30) with a bidirectional radio link (35) is provided for transmitting measurement data and the control signals between the mask (10) and the operating device (20) and an evaluation unit (40).

2. Device according to claim 1, **characterized in that** the operating device (20) generates the control signals for the display (12).

3. Device according to claim 1 or 2, **characterized by** a mirror system (15, 16; 15a) which is arranged within the mask (10) in such a way that it deflects the image appearing on the display (12) towards the viewer.

4. Device according to one of the preceding claims, **characterized in that** the evaluation unit (40) compares the tilt angle (α2) of the image relative to the mask (10) with the signal of the inclination sensor (18) when the image appears to be vertically aligned.

5. Device according to one of the preceding claims, **characterized in that** the inclination sensor (18) is designed as a three-dimensional acceleration sensor.

6. Device according to one of the preceding claims, **characterized in that** the image (7) is designed as a light pattern and/or as a light line.

7. Device according to one of the preceding claims, **characterized in that** the image (7) is configured as an image sequence or as a video.

8. Device according to one of the preceding claims, **characterized by** an image memory integrated in the mask (10).

9. Device according to one of the preceding claims, **characterized in that** a diffuser element (13) is arranged in front of the display.

10. Device according to one of the preceding claims, **characterized in that** the image shown on the display is free of edges, the image being in particular artificially noisy and/or having exclusively irregular structures.

11. Device according to any of the preceding claims, **characterized by** a 3-axis rotation rate sensor.

12. Method for determining the subjective visual vertical in persons, comprising the steps:
Displaying an image (7) on an electronic display within a mask (10) placed on a person; and
detecting the inclination of the head and/or an acceleration acting on the head with an inclination sensor (18);
wherein the image (7) is rotated about the viewing direction B, B' by means of control signals until it appears to the person as vertically aligned in space,
**characterized in**
**that** the mask (10) is opaque and is placed on the person in such a way that no light from the outside enters the interior (9) of the mask (10),
the inclination sensor (18) is integrated in the mask (10),
and the person manually adjusts the subjectively perceived vertical image position himself/herself with a portable operating device (20) while he/she is in any desired position,
and measurement data and the control signals are transmitted with a bidirectional radio link (35) between the mask (10) and the operating device (20) and an evaluation unit (40).

13. Method according to claim 12, **characterized in that** the person, in the case of a defined head tilt or acceleration, rotates the image (7) about the viewing direction B, B' into a position in which it appears to the person to be vertically aligned in space; and in the case of the image position thus set, the tilt angle of the image (7) relative to the mask (10) is detected and compared with the signal of the inclination sensor (18).

14. Method according to claim 12 or 13, **characterized in that** a light pattern and/or a light line is represented as image (7).

15. Method according to any one of claims 12 to 14, **characterized in that** an image sequence or a video is represented as image (7).

16. Method according to one of claims 12 to 15, **characterized in that** the person is accelerated during the measurement.

## Revendications

1. Dispositif pour tester la fonction otolithique chez les personnes, comprenant:
un masque (10) qui peut être placé sur une personne;
un capteur d'inclinaison (18) dont les signaux représentent l'inclinaison du masque (10) et/ou une accélération agissant sur le masque (10);
un écran électronique (12) pour afficher une image (7) à l'intérieur du masque (10),
un dispositif de commande (20) pour envoyer des signaux de commande au masque (10),
le dispositif de commande (20) comprenant des éléments de commande (22, 23) avec lesquels l'image (7) affichée sur l'écran (12) peut être pivotée dans le sens des aiguilles d'une montre ou dans le sens inverse des aiguilles d'une montre afin d'ajuster visuellement une position d'image alignée verticalement, **caractérisé en ce que**
le masque (10) est opaque et peut être placé sur la personne de manière étanche à la lumière,
le capteur d'inclinaison (18) est intégré dans le masque (10),
le dispositif de commande (20) est portable de sorte que la personne peut régler manuellement la position de l'image dans une position souhaitée, et
**en ce qu'**il est prévu un dispositif de transmission de données sans fil (30) avec une liaison radio bidirectionnelle (35) pour la transmission de données de mesure et des signaux de commande entre le masque (10) et le dispositif de commande (20) et une unité d'évaluation (40).

2. Dispositif selon la revendication 1, **caractérisé en ce que** le dispositif de commande (20) génère les signaux de commande pour l'écran (12).

3. Dispositif selon la revendication 1 ou 2, **caractérisé par** un système de miroirs (15, 16 ; 15a) disposé à l'intérieur du masque (10) de manière à dévier l'image apparaissant sur l'écran (12) vers l'observateur.

4. Dispositif selon l'une des revendications précédentes, **caractérisé en ce que** l'unité d'évaluation (40) compare l'angle d'inclinaison (α2) de l'image par rapport au masque (10) avec le signal du capteur d'inclinaison (18) quand l'image apparaît orientée verticalement.

5. Dispositif selon l'une des revendications précédentes, **caractérisé en ce que** le capteur d'inclinaison (18) est un capteur d'accélération tridimensionnel.

6. Dispositif selon l'une des revendications précédentes, **caractérisé en ce que** l'image (7) est arrangée sous forme de motif lumineux et/ou de ligne lumineuse.

7. Dispositif selon l'une des revendications précédentes, **caractérisé en ce que** l'image (7) est arrangée comme une séquence d'images ou comme une vidéo.

8. Dispositif selon l'une des revendications précédentes, **caractérisé par** une mémoire d'image intégrée dans le masque (10).

9. Dispositif selon l'une des revendications précédentes, **caractérisé en ce qu'**un élément diffuseur (13) est disposé devant l'écran.

10. Dispositif selon l'une des revendications précédentes, **caractérisé en ce que** l'image représentée sur l'écran est dépourvue d'arêtes, l'image étant notamment artificiellement bruitée et/ou présentant exclusivement des structures irrégulières.

11. Dispositif selon l'une des revendications précédentes, **caractérisé par** un capteur de vitesse de rotation à 3 axes.

12. Procédé de détermination de la verticale visuelle subjective chez des personnes, comprenant les étapes:
afficher une image (7) sur un écran électronique à l'intérieur d'un masque (10) placé sur une personne; et
détecter l'inclinaison de la tête et/ou une accélération agissant sur la tête avec un capteur d'inclinaison (18);
dans lequel l'image (7) est pivotée autour de la direction d'observation B, B' au moyen de signaux de commande jusqu'à ce qu'elle apparaisse à la personne comme étant orientée verticalement dans l'espace,
**caractérisé en ce que**
le masque (10) est opaque et est placé sur la personne de telle sorte que la lumière ne pénètre pas de l'extérieur à l'intérieur (9) du masque (10),
le capteur d'inclinaison (18) est intégré dans le masque (10),
et la personne règle elle-même manuellement la position verticale subjectivement perçue de l'image à l'aide d'un dispositif de commande portable (20), tout en étant dans une position souhaitée,
et des données de mesure et les signaux de commande sont transmis par une liaison radio bidirectionnelle (35) entre le masque (10) et le dispositif de commande (20) et une unité d'évaluation (40).

13. Procédé selon la revendication 12, **caractérisé en ce que** la personne, dans le cas d'une inclinaison ou d'une accélération définie de la tête, fait tourner l'image (7) autour de la direction d'observation B, B' dans une position dans laquelle elle apparaît à la personne comme étant alignée verticalement dans l'espace; et, avec la position d'image ainsi ajustée, l'angle d'inclinaison de l'image (7) par rapport au masque (10) est détecté et comparé au signal du capteur d'inclinaison (18).

14. Procédé selon la revendication 12 ou 13, **caractérisé en ce qu'**un motif lumineux et/ou une ligne lumineuse est représenté comme image (7).

15. Procédé selon l'une quelconque des revendications 12 à 14, **caractérisé en ce qu'**une séquence d'images ou une vidéo est représentée en tant qu'image (7).

16. Procédé selon l'une des revendications 12 à 15, **caractérisé en ce que** la personne est accélérée pendant la mesure.
